# EUROPEAN PATENT SPECIFICATION

(11) **EP 2 636 371 B1**
(45) Date of publication and mention of the grant of the patent: **19.10.2016**
(21) Application number: 12158835.4
(22) Date of filing: 09.03.2012
(51) Int. Cl.: A61B 5/11, A61B 7/00, G06K 9/00

(54) **Activity classification**
Aktivitätsklassifizierung
Classification d'activité

(43) Date of publication of application: 11.09.2013
(73) Proprietor: Sony Mobile Communications AB, 221 88 Lund (SE)
(72) Inventor: Thörn, Karl Ola, 216 12 Limhamn (SE); Mårtensson, Linus, 246 50 Löddeköpinge (SE); Bengtsson, Henrik, 227 36 Lund (SE); Aronsson, Pär-Andersson, 214 24 Malmö (SE); Jonsson, Håkan, 245 65 Hjärup (SE)
(74) Representative: Awapatent AB

(56) References cited:
- WO-A1-2009/153681
- WO-A1-2011/141916
- WO-A2-2008/098943
- US-A1- 2008 269 646
- US-A1- 2011 082 698

## Description

### TECHNICAL FIELD

The present invention relates to method and devices for classifying activity of a user, especially using sound information.

### BACKGROUND

With the rapid development of the mobile terminals such as mobile phones, more and more functionalities are incorporated inside the terminal. One feature is to detect motion of the terminal and thereby the motion and activity of the user.

US 2008/0269646 discloses a system and method for detecting swallowing activity.

WO 2011/141916 discloses a device and method for monitoring and analyzing breathing sounds.

WO 2009/153681 discloses acoustical patient monitoring using a sound classifier and a microphone.

WO 2008/098943 discloses arrangement and method to wake up a sleeping subject at an advantageous time instant associated with natural arousal.

Activity recognition, i.e. classifying how a user is moving, e.g. sitting, running, walking, riding a car etc., is currently done mainly using accelerometer sensors and in some cases location sensors or video. Activity recognition in handsets is a problem since it may consume a lot of power and also has limited accuracy. This invention tries to solve this by using body microphones to capture sound of vibrations transported through the user's body to improve accuracy and/or reduce power consumption. It improves accuracy compared to using only accelerometer or microphones recording external (non-body) sounds.

### SUMMARY

The present invention, which is defined in claims 1 - 14, provides a solution to aforementioned problem by using body attached microphones to capture sound of vibrations transported through the user's body to improve accuracy and/or reduce power consumption.

### BRIEF DESCRIPTION OF THE DRAWINGS

Reference is made to the attached drawings, wherein elements having the same reference number designation may represent like elements throughout.
Fig. 1 is a diagram of an exemplary arrangement in which methods and systems described herein may be implemented;
Fig. 2 is a diagram of an exemplary system in which methods and systems described herein may be implemented;
Fig. 3 is a diagram of an exemplary sensor device according to one embodiment of the invention; and
Fig. 4 is a diagram over the steps of an exemplary embodiment according to the invention.

### DETAILED DESCRIPTION

The following detailed description refers to the accompanying drawings. The same reference numbers in different drawings may identify the same or similar elements.
The term "image," as used herein, may refer to a digital or an analog representation of visual information (e.g., a picture, a video, a photograph, animations, etc.)
The term "audio" as used herein, may include may refer to a digital or an analog representation of audio information (e.g., a recorded voice, a song, an audio book, etc.)

Also, the following detailed description does not limit the invention. Instead, the scope of the invention is defined by the appended claims and equivalents.

The basic idea of the invention is to record sound waves internally transported through the body of a user itself. This makes it suitable to also recognize activities that do not generate distinct external sounds, e.g. walking or running. It also makes it less susceptible to ambient noise and thus provides higher accuracy.

The microphone(s) can be placed, e.g. using a holder on the body of a user. The microphones may be provided facing the body and in direct contact with the skin. The activity classification itself can be done in a sensor and then communicated to the terminal to be used in applications. The sound type detection may be carried on in a lower level feature detection, which is then communicated to the terminal where the actual activity classification is done.

The audio and accelerometer and audio data is preprocessed to extract features and then fed to the classifier, which can be an assembly of classifiers, which then generates a classification. The specific classification method used, e.g. bayesian, neural networks etc, is an implementation detail.

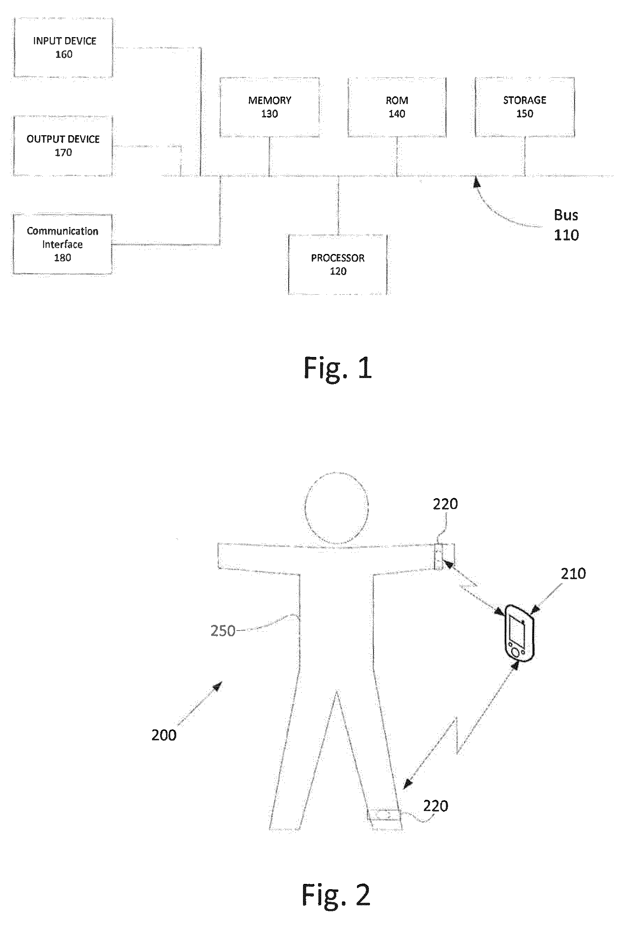

Fig. 1 is a diagram of an exemplary arrangement 100 (internal) in which methods and systems described herein may be implemented. Arrangement 100 may include a bus 110, a processor 120, a memory 130, a read only memory (ROM) 140, a storage device 150, an input device 160, an output device 170, and a communication interface 180. Bus 110 permits communication among the components of arrangement 100. Arrangement 100 may also include one or more power supplies (not shown). One skilled in the art would recognize that arrangement 100 may be configured in a number of other ways and may include other or different elements.

Processor 120 may include any type of processor or microprocessor that interprets and executes instructions. Processor 120 may also include logic that is able to decode media, such as audio and audio files, etc., and generate output to, for example, a speaker, a display, etc. Memory 130 may include a random access memory (RAM) or another dynamic storage device that stores information and instructions for execution by processor 120. Memory 130 may also be used to store temporary variables or other intermediate information during execution of instructions by processor 120.

ROM 140 may include a conventional ROM device and/or another static storage device that stores static information and instructions for processor 120. Storage device 150 may include a flash memory (e.g., an electrically erasable programmable read only memory (EEPROM)) device for storing information and instructions.

Input device 160 may include one or more conventional mechanisms that permit a user to input information to the arrangement 100, such as a keyboard, a keypad, a directional pad, a mouse, a pen, voice recognition, a touch-screen and/or biometric mechanisms, etc. Output device 170 may include one or more conventional mechanisms that output information to the user, including a display, a printer, one or more speakers, etc. Communication interface 180 may include any transceiver-like mechanism that enables arrangement 100 to communicate with other devices and/or systems. For example, communication interface 180 may include a modem or an Ethernet interface to a LAN.

Alternatively, or additionally, communication interface 180 may include other mechanisms for communicating via a network, such as a wireless network. For example, communication interface may include a radio frequency (RF) transmitter and receiver and one or more antennas for transmitting and receiving RF data.

Arrangement 100, consistent with the invention, provides a platform through which audible information and motion information may be interpreted to activity information. Arrangement 100 may also display information associated with the activity to the user of arrangement 100 in a graphical format or provided to a third part system. According to an exemplary implementation, arrangement 100 may perform various processes in response to processor 120 executing sequences of instructions contained in memory 130. Such instructions may be read into memory 130 from another computer-readable medium, such as storage device 150, or from a separate device via communication interface 180. It should be understood that a computer-readable medium may include one or more memory devices or carrier waves. Execution of the sequences of instructions contained in memory 130 causes processor 120 to perform the acts that will be described hereafter. In alternative embodiments, hard-wired circuitry may be used in place of or in combination with software instructions to implement aspects consistent with the invention. Thus, the invention is not limited to any specific combination of hardware circuitry and software.

Fig. 2 illustrates a system 200 according to the invention. The system 200 comprises a mobile terminal 210, such as a mobile radio phone, and a number of sensors 220 attached to a user 250.

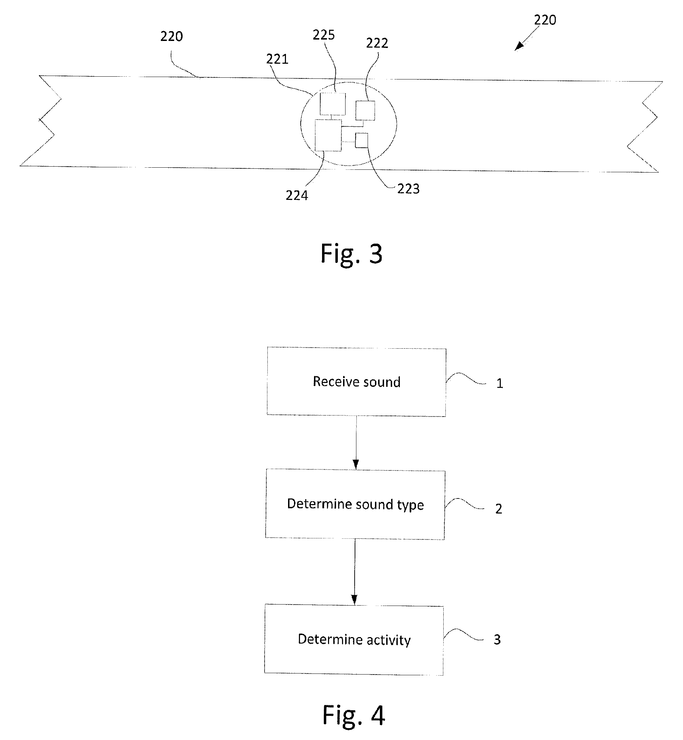

The mobile terminal 210 may comprise an arrangement according to Fig. 1 as described earlier. The sensor 220 Is described in more detail in the embodiment of Fig. 3.

Fig. 3 is a diagram of an exemplary embodiment of a sensor 220. The sensor 220 comprises a housing 221, inside which a microphone 222, a motion sensor 223, a controller 224 and a transceiver 225 are arranged. A power source and other electrical portions, such as memory, may also be arranged inside the housing but are not illustrated for clarity reasons.

The housing 221 may be provided on an attachment portion 225, such as strap or band. The attachment portion 225 allows the sensor portion to be attached to a body part of user. The attachment portion may comprise VELCRO fastening band, or any other type of fastening, which In one embodiment may allow the user to attach the sensor 220 to a body part, such as wrist, ankle, chest etc. The sensor may also be integrated in or attached to a watch, clothing, socks, gloves, etc.

The microphone 222, in one embodiment facing the skin of the user, records sound waves internally transported through the body of the user itself, which allows recognizing activities that do not generate distinct external sounds, e.g. body activities such as running or walking. It also makes it less susceptible to ambient noise and thus provides higher accuracy.

The motion sensor 223, such as accelerometer, gyro etc., allows detecting movement of the user.

In one embodiment, the sensor 220 may only record sound, i.e. only comprise microphone or in lack of motion only use microphone. In one embodiment both the microphone and the motion sensor are in MEMS (Microelectromechanical systems).

The control 224 receives signals from the microphone 222 and motion sensor 223 and, depending on the configuration, may process the signals or transmit them to the mobile terminal. The controller 224 may include any type of processor or microprocessor that interprets and executes instructions. The controller may also include logic that is able to decode media, such as audio and audio files, etc., and generate output to, for example, a speaker, a display, etc. The controller may also include onboard memory for storing information and instructions for execution by the controller.

The transceiver 225, which may include an antenna (not shown), may use wireless communication including radio signals, such as Bluetooth, Wi-Fi, or IR or wired communication, mainly to transmit signals to the terminal 210 (or other devices).

With reference now to Figs. 2, 3 and 4, in operation, according to one embodiment, the microphone 222 in contact with user 250 skin of the sensor 220 receives (1) sound waves, which are converted to electrical signals and provided to the controller 224. If the sensor 220 is used to classify activity, parts of arrangement 100 may be Incorporated therein. The controller may store the sound signal. A memory may also store a sound database, which includes a plurality of sound types and a plurality of attributes associated with each sound type. Each attribute may have a predefined value and each sound type may be associated with each attribute In accordance with, e.g. Bayesian's rule, such that a conditional probability of each sound type Is defined for an occurrence of each attribute. The attributes may consist of: histogram features, linear predictive coding, cepstral coefficients, short-time Fourier transform, timbre, zero-crossing rate, short-time energy, root-mean-square energy, high/low feature value ratio, spectrum centroid, spectrum spread, spectral roll-off frequency, etc. Other determination methods using neural networks, or the like, comparison methods may also be used to determine the type of sound.

A more accurate classification may be obtained using the signal from the motion detector 223. Different motions, e.g. walking, running, dancing etc. have different movement characteristics.

The sensor 222 may also be provided with other detectors, e.g. pulsimeter, heartbeat meter, temperature meter, etc.

When the type of sound Is determined (2), the activity classification, Irrespective of where (sensor, terminal, network) It Is carried out, may comprise comparing the sound type data (and motion data and other relevant data) with stored data in a database, or use Bayesian, neural network methods to classify (3) the activity. The classification may be carried out in the sensor or the data is provided to the mobile terminal or a network device for classification.

In one example, the user may have two sensors, as in Fig. 2, one attached to wrist and ankle. During a walk, the motion sensor has a lower movement pace and the microphones pick up sound e.g. from the ankle and wrist. The vibrations during the walk are lower, If the user starts running, the vibrations, especially from the ankle microphone will Increase and also the movement pace.

It should be noted that the word "comprising" does not exclude the presence of other elements or steps than those listed and the words "a" or "an" preceding an element do not exclude the presence of a plurality of such elements. It should further be noted that any reference signs do not limit the scope of the claims, that the invention may be implemented at least in part by means of both hardware and software, and that several "means", "units" or "devices" may be represented by the same item of hardware.
A "device" as the term is used herein, is to be broadly interpreted to include a radiotelephone having ability for receiving and processing sound and other data. The device may also be a sound recorder, global positioning system (GPS) receiver; a personal communications system (PCS) terminal that may combine a cellular radiotelephone with data processing; a personal digital assistant (PDA); a laptop; a camera (e.g., video and/or still image camera) having communication ability; and any other computation or communication device capable of transceiving, such as a personal computer, a home entertainment system, a television, etc.

The various embodiments of the present invention described herein is described in the general context of method steps or processes, which may be implemented in one embodiment by a computer program product, embodied in a computer-readable medium, including computer-executable instructions, such as program code, executed by computers in networked environments. A computer-readable medium may include removable and non-removable storage devices including, but not limited to, Read Only Memory (ROM), Random Access Memory (RAM), compact discs (CDs), digital versatile discs (DVD), etc. Generally, program modules may include routines, programs, objects, components, data structures, etc. that perform particular tasks or implement particular abstract data types. Computer-executable instructions, associated data structures, and program modules represent examples of program code for executing steps of the methods disclosed herein. The particular sequence of such executable instructions or associated data structures represents examples of corresponding acts for implementing the functions described in such steps or processes.

Software and web implementations of various embodiments of the present invention can be accomplished with standard programming techniques with rule-based logic and other logic to accomplish various database searching steps or processes, correlation steps or processes, comparison steps or processes and decision steps or processes. It should be noted that the words "component" and "module," as used herein and in the following claims, is intended to encompass implementations using one or more lines of software code, and/or hardware implementations, and/or equipment for receiving manual inputs.

The foregoing description of embodiments of the present invention, have been presented for purposes of illustration and description. The foregoing description is not intended to be exhaustive or to limit embodiments of the present invention to the precise form disclosed, and modifications and variations are possible in light of the above teachings or may be acquired from practice of various embodiments of the present invention. The embodiments discussed herein were chosen and described in order to explain the principles and the nature of various embodiments of the present invention and its practical application to enable one skilled in the art to utilize the present invention in various embodiments and with various modifications as are suited to the particular use contemplated. The features of the embodiments described herein may be combined in all possible combinations of methods, apparatus, modules, systems, and computer program products.

Other solutions, uses, objectives, and functions within the scope of the invention as claimed in the below described patent claims should be apparent for the person skilled in the art.

## Claims

1. A method for classifying a type of a motion activity of a person, the activity pertaining to walking or running, based on sound, the method comprising:
• receiving (1) a sound signal from a sensor (222), said signal corresponding to a sound of vibrations internally transported through body of the person,
• determining type of said sound based on said sound signal, and
• determining said motion activity type, being walking or running, based on said type of sound generated due to said activity.

2. The method of claim 1, wherein said sensor (222) is a microphone facing body of a user (250).

3. The method according to any or previous claims, wherein said sensor further comprises a motion detector (223).

4. The method according to any or previous claims, further comprising comparing said sound signal with a number of sound signals stored in a memory, which includes a plurality of sound types and a plurality of attributes associated with each sound type.

5. The method according to any of previous claims, further comprising using Bayesian rules or neural network for classifying the motion activity.

6. The method according to claim 4, wherein each attribute comprises a predefined value and each sound type is associated with each attribute.

7. The method according to claim 5, wherein each sound type is associated with each attribute in accordance with the Bayesian rules, such that a conditional probability of each sound type is defined for an occurrence of each attribute.

8. The method according claim 4, wherein said attributes consist of one or several of: histogram features, linear predictive coding, cepstral coefficients, short-time Fourier transform, timbre, zero-crossing rate, short-time energy, root-mean-square energy, high/low feature value ratio, spectrum centroid, spectrum spread, or spectral roll-off frequency.

9. A device (100) for classifying a type of motion activity of a person (250), the activity pertaining to walking or running, based on sound generated by the activity, the device (100) comprising: a receiver for receiving a sound signal from a sensor (220), and a controller (120), **characterised in that** said sensor is arranged to receive a sound of vibrations transported through body of the person, and output a sound signal corresponding to said sound wave and the controller (120) is configured to process said sound signal and determine type of sound based on said sound signal, and determine said motion activity type, being walking or running, based on said type of sound.

10. The device of claim 9, wherein said sound signals are received from one or several microphones attached to said person.

11. The device according to any of claims 9-10, further receiving motion data from one or several motion detectors.

12. The device according to any of claims 9-11, wherein said controller is configured to compare said sound signal with a number of sound signals stored in a memory, which includes a plurality of sound types and a plurality of attributes associated with each sound type, each attribute comprising a predefined value and each sound type is associated with each attribute, each sound type is associated with each attribute in accordance with Bayesian's rule, such that a conditional probability of each sound type is defined for an occurrence of each attribute.

13. The device according claim 12, wherein said attributes consist of one or several of: histogram features, linear predictive coding, cepstral coefficients, short-time Fourier transform, timbre, zero-crossing rate, short-time energy, root-mean-square energy, high/low feature value ratio, spectrum centroid, spectrum spread, or spectral roll-off frequency.

14. A mobile communication terminal comprising a device according to any of claims 9-13.

## Patentansprüche

1. Verfahren zum Klassifizieren einer Art von Bewegungsaktivität einer Person basierend auf Schall, wobei die Aktivität Gehen oder Laufen betrifft, wobei das Verfahren Folgendes umfasst:
• Empfangen (1) eines Schallsignals von einem Sensor (222), wobei das Schallsignal einem Schall von Vibrationen entspricht, der im Inneren durch den Körper der Person transportiert wird,
• Bestimmen der Art des Schalls basierend auf dem Schallsignal, und
• Bestimmen der Bewegungsaktivitätsart, die Gehen und Laufen ist, basierend auf der Art von Schall, der durch diese Aktivität erzeugt wird.

2. Verfahren nach Anspruch 1, wobei der Sensor (222) ein Mikrofon ist, das dem Körper eines Benutzers (250) zugewandt ist.

3. Verfahren nach einem der vorhergehenden Ansprüche, wobei der Sensor ferner einen Bewegungsmelder (223) umfasst.

4. Verfahren nach einem der vorhergehenden Ansprüche, das ferner Vergleichen des Schallsignals mit einer Reihe von Schallsignalen, die in einem Speicher gespeichert sind, der mehrere Schallsignale und mehrere Attribute, die mit jedem Schallsignal assoziiert sind, beinhaltet, umfasst.

5. Verfahren nach einem der vorhergehenden Ansprüche, das ferner Verwenden bayesscher Regeln oder eines neuronalen Netzwerks zum Klassifizieren der Bewegungsaktivität umfasst.

6. Verfahren nach Anspruch 4, wobei jedes Attribut einen vordefinierten Wert umfasst und jede Schallart mit jedem Attribut assoziiert ist.

7. Verfahren nach Anspruch 5, wobei jede Schallart mit jedem Attribut gemäß bayesscher Regeln assoziiert ist, sodass eine bedingte Wahrscheinlichkeit von jeder Schallart für ein Auftreten jedes Attributs definiert ist.

8. Verfahren nach Anspruch 4, wobei die Attribute aus einem oder mehreren der Folgenden bestehen: Histogrammmerkmale, Linear-Predicitve-Coding, Cepstrum-Koeffizienten, Kurzzeit-FourierTransformation, Timbre, Nulldurchgangsrate, Kurzzeitenergie, Root-Mean-Square-Energie, hohes/niedriges Merkmalswertverhältnis, Spektrum-Zentroid, Spektrumsspreizung oder spektrale Rolloff-Frequenz.

9. Vorrichtung (100) zum Klassifizieren einer Bewegungsaktivität einer Person (250) basierend auf Schall, der durch die Aktivität erzeugt wird, wobei die Aktivität Gehen oder Laufen betrifft, wobei die Vorrichtung (100) Folgendes umfasst: einen Empfänger zum Empfangen eines Schallsignals von einem Sensor (220) und eine Steuerung (120), **dadurch gekennzeichnet, dass** der Sensor angeordnet ist, um einen Schall von Vibrationen, der durch den Körper der Person transportiert wird, zu empfangen und ein Schallsignal, das der Schallwelle entspricht, auszugeben, und wobei die Steuerung (120) konfiguriert ist, das Schallsignal zu verarbeiten und eine Art von Schall basierend auf dem Schallsignal zu bestimmen und die Bewegungsaktivitätsart, die Gehen oder Laufen ist, basierend auf der Art von Schall zu bestimmen.

10. Vorrichtung nach Anspruch 9, wobei die Schallsignale von einem oder mehreren Mikrofonen, die an dem Körper der Person befestigt sind, empfangen werden.

11. Vorrichtung nach einem der Ansprüche 9 - 10, die ferner Bewegungsdaten von einem oder mehreren Bewegungsdetektoren empfängt.

12. Vorrichtung nach einem der Ansprüche 9 - 11, wobei die Steuerung konfiguriert ist, das Schallsignal mit einer Anzahl von Schallsignalen zu vergleichen, die in einem Speicher gespeichert sind, der mehrere Schallarten und mehrere Attribute, die mit jeder Schallart assoziiert sind, umfasst, wobei jedes Attribut einen vordefinierten Wert umfasst und jede Schallart mit jedem Attribut assoziiert ist, wobei jede Schallart mit jedem Attribut gemäß bayesschen Regeln assoziiert ist, sodass eine bedingte Wahrscheinlichkeit jeder Schallart für ein Auftreten jeden Attributs definiert ist.

13. Vorrichtung nach Anspruch 12, wobei die Attribute aus einem oder mehreren der Folgenden bestehen: Histogrammmerkmale, Linear-Predicitve-Coding, Cepstrum-Koeffizienten, Kurzzeit-FourierTransformation, Timbre, Nulldurchgangsrate, Kurzzeitenergie, Root-Mean-Square-Energie, hohes/niedriges Merkmalswertverhältnis, Spektrum-Zentroid, Spektrumsspreizung oder spektrale Rolloff-Frequenz.

14. Mobiler Kommunikationsterminal, der eine Vorrichtung nach einem der Ansprüche 9 - 13 umfasst.

## Revendications

1. Procédé de classification d'un type d'activité de mouvement d'une personne, l'activité se rapportant à la marche ou la course, sur la base de sons, le procédé comprenant de :
• recevoir (1) un signal sonore provenant d'un capteur (222), ledit signal correspondant à un son de vibrations transportées intérieurement à travers le corps de la personne,
• déterminer un type dudit son sur la base dudit signal sonore, et
• déterminer ledit type d'activité de mouvement, étant la marche ou la course, sur la base dudit type de son généré du fait de l'activité.

2. Procédé selon la revendication 1, dans lequel ledit capteur (222) est un corps faisant face à un microphone d'un utilisateur (250).

3. Procédé selon une quelconque des revendications précédentes, dans lequel ledit capteur comprend en outre un détecteur de mouvement (223).

4. Procédé selon une quelconque des revendications précédentes, comprenant en outre de comparer ledit signal sonore avec une pluralité de signaux sonores mémorisés dans une mémoire, qui inclut une pluralité de types de son et une pluralité d'attributs associés à chaque type de son.

5. Procédé selon une quelconque des revendications précédentes, comprenant en outre d'utiliser des règles Bayésiennes ou un réseau neural pour classifier l'activité de mouvement.

6. Procédé selon la revendication 4, dans lequel chaque attribut comprend une valeur prédéfinie et chaque type de son est associé à chaque attribut.

7. Procédé selon la revendication 5, dans lequel chaque type de son est associé à chaque attribut conformément aux règles Bayésiennes, de sorte qu'une probabilité conditionnelle de chaque type de son soit définie pour une occurrence de chaque attribut.

8. Procédé selon la revendication 4, dans lequel lesdits attributs sont constitués d'un ou plusieurs de : caractéristiques d'histogramme, codage prédictif linéaire, coefficients cepstraux, transformée de Fourier à court terme, timbre, taux de passage de zéro, énergie à court terme, énergie de racine carrée moyenne, rapport de valeur caractéristique haut/bas, centroïde de spectre, étalement de spectre ou fréquence de coupure spectrale.

9. Dispositif (100) pour classifier un type d'activité de mouvement d'une personne (250), l'activité se rapportant à la marche ou la course, sur la base des sons générés par l'activité, le dispositif (100) comprenant : un récepteur pour recevoir un signal sonore provenant d'un capteur (220) et un contrôleur (120), **caractérisé en ce que** ledit capteur est agencé pour recevoir un son de vibrations transportées intérieurement à travers le corps de la personne, et délivrer un signal sonore correspondant à ladite onde sonore et le contrôleur (120) est configuré pour traiter ledit signal sonore et déterminer le type de son sur la base dudit signal sonore, et déterminer ledit type d'activité de mouvement, étant la marche ou la course, sur la base dudit type de son.

10. Dispositif selon la revendication 9, dans lequel lesdits signaux sonores sont reçus à partir d'un ou plusieurs microphones fixés à la personne.

11. Dispositif selon une quelconque des revendications 9 - 10, comprenant en outre de recevoir des données de mouvement provenant d'un ou plusieurs détecteurs de mouvement.

12. Dispositif selon une quelconque des revendications 9 - 11, dans lequel ledit contrôleur est configuré pour comparer ledit signal sonore avec une pluralité de signaux sonores mémorisés dans une mémoire, qui inclut une pluralité de types de son et une pluralité d'attributs associés à chaque type de son, chaque attribut comprenant une valeur prédéfinie et chaque type de son est associé à chaque attribut, chaque type de son est associé à chaque attribut conformément à des règles Bayésiennes, de sorte qu'une probabilité conditionnelle de chaque type de son soit définie pour une occurrence de chaque attribut.

13. Dispositif selon la revendication 12, dans lequel lesdits attributs sont constitués d'un ou plusieurs de : caractéristiques d'histogramme, codage prédictif linéaire, coefficients cepstraux, transformée de Fourier à court terme, timbre, taux de passage de zéro, énergie à court terme, énergie de racine carrée moyenne, rapport de valeur caractéristique haut/bas, centroïde de spectre, étalement de spectre ou fréquence de coupure spectrale.

14. Terminal de communication mobile comprenant un dispositif selon une quelconque des revendications 9 - 13.
